(19) Europäisches Patentamt / European Patent Office / Office européen des brevets

(11) **EP 4 553 040 A1**

(12) **EUROPEAN PATENT APPLICATION**

(43) Date of publication:
14.05.2025 Bulletin 2025/20

(51) International Patent Classification (IPC):
*C01B 13/10* *(2006.01)*

(21) Application number: 24210307.5

(22) Date of filing: 31.10.2024

(52) Cooperative Patent Classification (CPC):
**C01B 13/10;** C01B 2201/90

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **13.11.2023 IT 202300023997**

(71) Applicant: **MULTIOSSIGEN S.P.A.
24020 Gorle (BG) (IT)**

(72) Inventor: **FRANZINI, Marianno
24129 Bergamo (BG) (IT)**

(74) Representative: **Gatti, Enrico et al
Giambrocono & C. S.p.A.
Via E. Zambianchi, 3
24121 Bergamo (IT)**

(54) **APPARATUS FOR GENERATING OZONE**

(57) Apparatus for generating ozone comprising: an ozone generator (15); a device (21) for collecting the ozone generated; a device (24) for discharging the excess ozone; characterized by comprising a first measuring system of the ozone concentration comprising: a first measuring device (20) of a first ozone concentration; a first pressure measuring device (14) of the gas entering said first measuring device (20) of a first ozone concentration to supply a pressure measurement; a first flow rate measuring device (12) of the gas entering said first measuring device (20) of the ozone concentration to supply a flow rate measurement; a control system (30, 31) of said apparatus; said control system (30, 31) calculates a second ozone concentration based on said first ozone concentration, on said pressure measurement and on said flow rate measurement.

*Fig. 1*

Processed by Luminess, 75001 PARIS (FR)

## Description

The present invention refers to an apparatus for generating ozone.

For over twenty years ozone has been successfully used in various areas of the health sector. Its applications in the field of medicine mainly concern two aspects: the use of ozone as natural alternative to conventional chemical disinfection and sanitizing processes and Oxygen-Ozone Therapy for treating patients suffering from various diseases.

Medical reports on the effective use of ozone in the treatment of various diseases have shown that this gas has several beneficial effects. Due to its oxidative capacity, ozone kills bacteria by attacking their protective membrane. Ozone is also capable of penetrating the internal structure of the virus, preventing its replication.

The object of the present invention is to provide an apparatus for generating ozone that can be controlled with extreme precision.

In accordance with the present invention, these and other objects are achieved by an apparatus for generating ozone comprising: an ozone generator; a device for collecting the ozone generated; a device for discharging the ozone; characterized by comprising a first system for measuring the ozone concentration comprising: a first measuring device of a first ozone concentration; a first pressure measuring device of gas entering the first measuring device of a first ozone concentration to supply a pressure measurement; a first flow rate measuring device of the gas entering the first measuring device of the ozone concentration to supply a flow rate measurement; a control system of the apparatus; the control system calculates a second ozone concentration based on the first ozone concentration, on the pressure measurement and on the flow rate measurement; and in that it comprises a second system for measuring the ozone concentration comprising a second measuring device of a third ozone concentration located downstream of the device for discharging the ozone; the control system compares the value of the third ozone concentration with a preset value and if the value of the third ozone concentration exceeds the preset value it blocks the apparatus.

Further features of the invention are described in the dependent claims.

The advantages of this solution compared to the solutions of the prior art solutions are various.

The concentration of ozone is measured using an insulated and sealed cuvette, particularly compact, due to the use of ultraviolet LEDs rather than lamps of larger size. Therefore, the whole apparatus is of limited size.

Measurement of the ozone concentration is very accurate and precise, due to the measurement, as well as the ozone concentration, also of the pressure and of the gas flow entering the cuvette, detected by respective sensors.

Consequently, the operator has a greater guarantee of the ozone concentration used.

Moreover, a secondary ozone measuring device is present, the function of which is to analyse the ozone concentration discharged from the apparatus to check that the emissions fall within the admissible levels according to the current regulations, and if they exceed these limits to force machine stoppage with a service request.

A control board with a user interface is capable of controlling the apparatus and producing ozone according to previously stored protocols that the user can select by scrolling through a library displayed on the interface.

Again via the control board, the user can manually vary the parameters set.

The features and the advantages of the present invention will be apparent from the following detailed description of a practical embodiment thereof, illustrated by way of non-limiting example in the accompanying drawings, wherein:

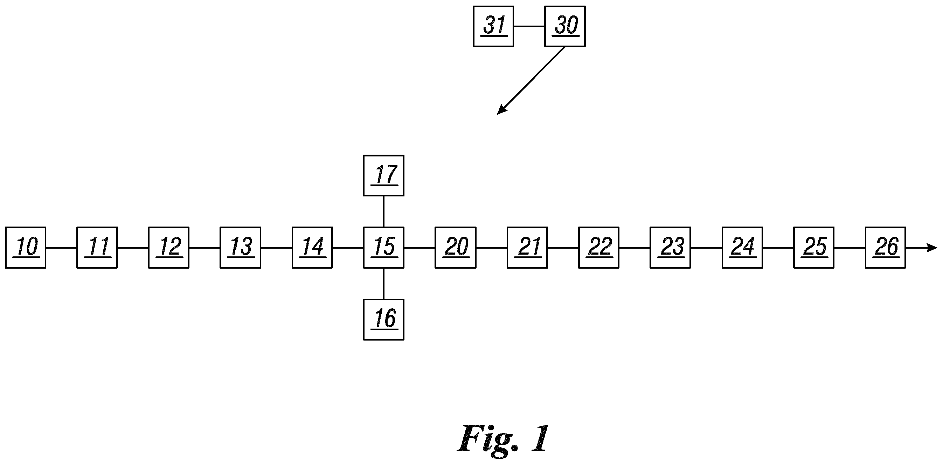

Fig. 1 schematically shows an apparatus for generating ozone, in accordance with the present invention,

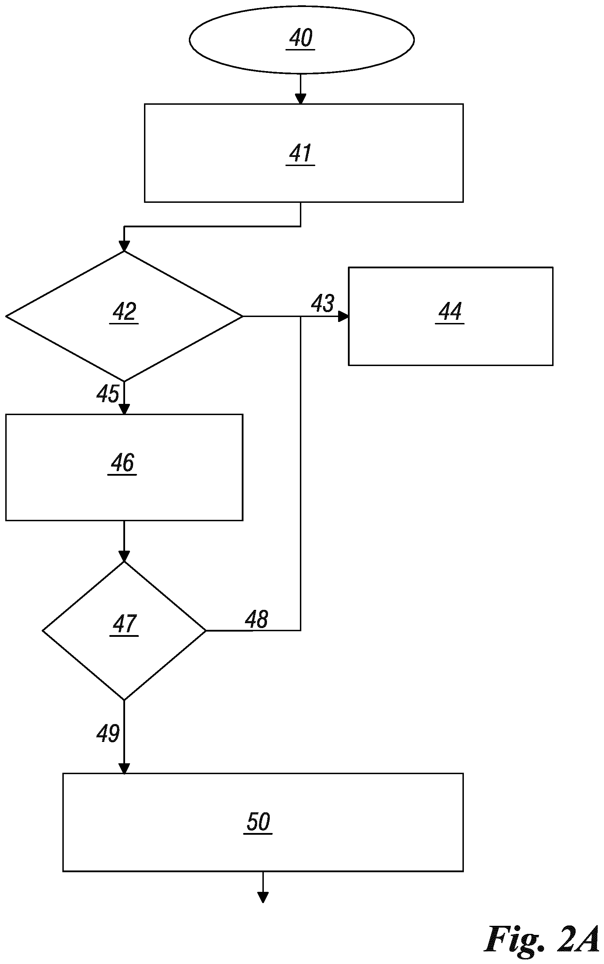

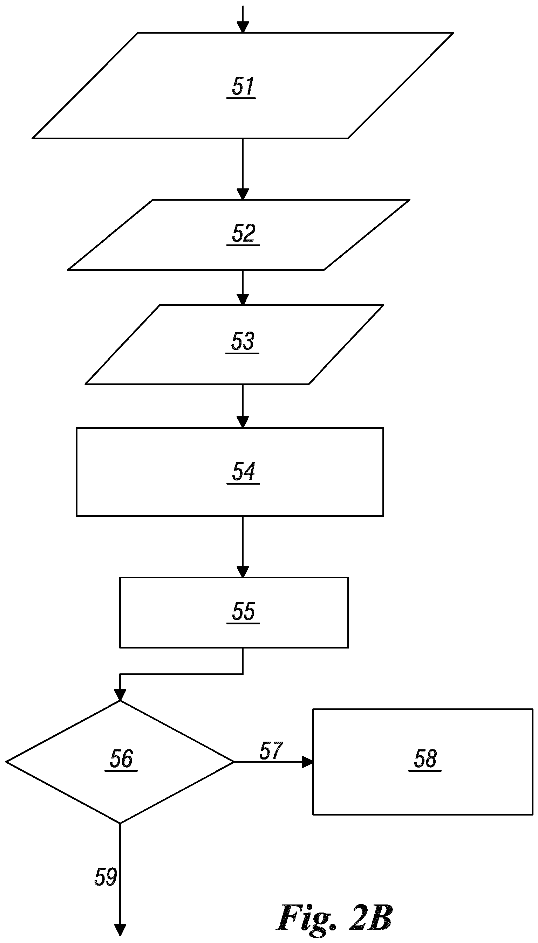

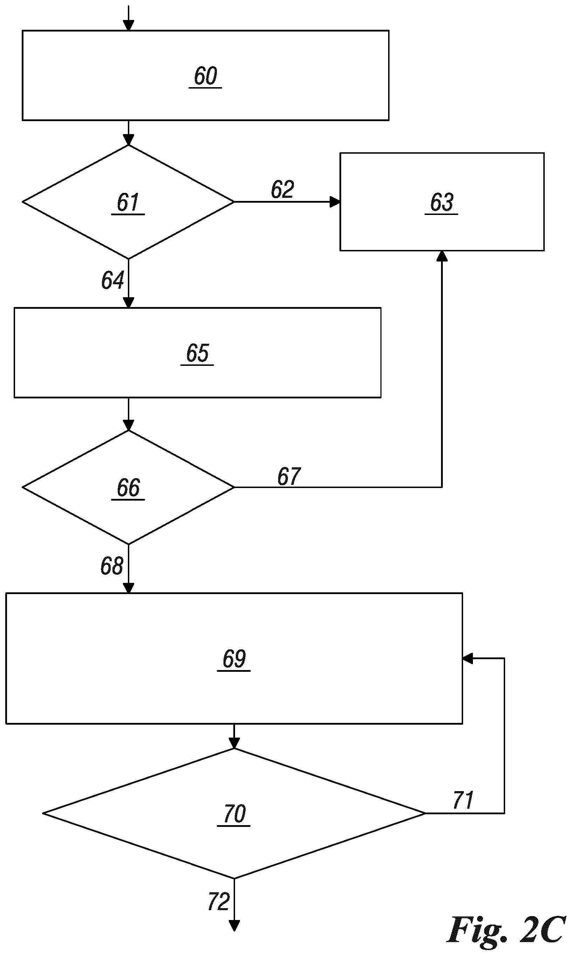

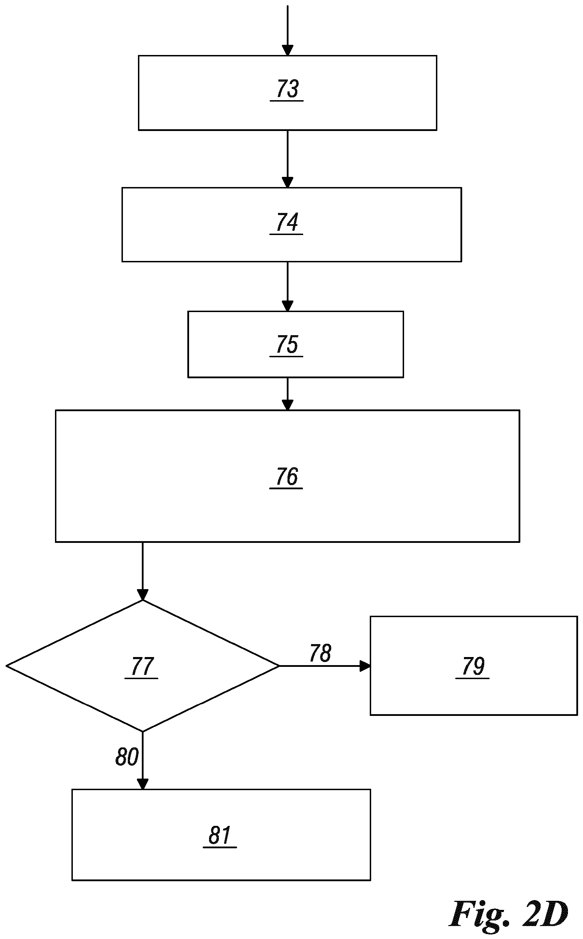

Figs. 2A, 2B, 2C and 2D show the flow chart with which the control board of the apparatus, in accordance with the present invention, operates.

With reference to the accompanying figures, an apparatus for generating ozone, in accordance with the present invention, comprises an oxygen cylinder 10 followed by a proportional solenoid valve 11 for regulating the flow rate of oxygen, followed by a flow meter 12 for measuring the flow rate measured in standard litres per minute (SLM), then by a solenoid valve 13 for opening/closing the flow of oxygen, and by a first sensor 14 for measuring the pressure.

Then, an ozone generator 15 is present, preferably of the type with pipes, with relevant high voltage generator 16 and with a system 17 for cooling the pipes, preferably via fans.

The ozone generator 15 is configured to supply ozone in the range from 0 to 100µg/ml.

A first measuring device 20 of the ozone concentration is located downstream of the ozone generator 15.

The first measuring device 20 comprises a cuvette which is parallelepiped shaped and made of aluminium treated inside the chamber, and which measures the ozone concentration by measuring the absorption of ultraviolet radiation (UV) by the ozone.

La cuvette has an ozone inlet and an ozone outlet and laterally thereto has two opposite portions made of borosilicate (transparent) for analysing the ozone.

An LED, which emits in the ultraviolet at a wavelength of 275/280nm, is associated with one borosilicate portion.

An ultraviolet sensor is associated with the other borosilicate portion.

The first measuring system 20 allows precise measurement of the concentrations of ozone in a wide range from 0 to 100µg/ml.

The cuvette is particularly compact and of small size due to the use of an ultraviolet LED instead of conventional ultraviolet lamps of larger size.

A station 21 for collecting ozone, composed of a three-way valve that allows the user to collect the required amount of ozone at the required concentration, is located downstream of the first measuring device 20.

There is then provided a second flow meter 22 for measuring the flow rate followed by a second sensor 23 for measuring the pressure. This is followed by a device 24 for discharging the ozone 24. This device 24, which is configured to discharge the excess ozone, comprises a catalyst 24 including activated carbon filters for ozone abatement.

There is then provided a second measuring device 25 of the ozone concentration by means of cuvette, similar to the first measuring device 20 of the ozone concentration, to measure the presence of ozone exceeding the permissible limits for emission into the atmosphere and block operation of the apparatus if the permissible limits are exceeded and subsequently invite the user to contact the support service.

The second measuring device 25 is followed by an opening/closing solenoid valve 26 to prevent any releases of ozone.

All the elements of the apparatus are controlled by a control board 30 inside the apparatus, which operates through a suitable program.

The control board 30 includes an interface 31 with monitor, keyboard and mouse (or touch screen) and everything else necessary for the operator.

Each element belonging to the apparatus is controlled by the control board 30.

The control board 30 manages the commands, the information received from the interface 31 translating them into instructions for executing functions of the apparatus. Using algorithms it processes the data received from the sensors to send information on the ozone concentrations, and on the operating status of the apparatus.

In particular, the control board 30 proposes protocols for different therapies on the interface 31.

The user can select the treatment protocol to use and the apparatus is capable of automatically supplying the concentration requested by the protocol and the flow rate by regulating the proportional solenoid valve 11. The user can vary, autonomously, the value of the concentration and of the flow rate, increasing them or decreasing them.

The ozone concentration is determined via the measurements obtained from the cuvettes 20 and 25 by means of the Beer-Lambert law which is an empirical relationship that correlates the amount of light absorbed by a material to the concentration and to the thickness of the material passed through.

To obtain a more precise measurement of the ozone concentration, measurements obtained by the flow meters 12 and 22 and by the pressure sensors 14 and 23, which provide multiplicative factors that correct the Beer-Lambert equation, are also used.

The calculation operations are executed by the control board 30 in accordance with the following relationship.

$$C = -(10^6/\ (s*l))*(M/NA)*\ln(L/l_0)*(T_c/T_0)*(Prc/Pr_0)*Zcp$$

where

C = ozone concentration (Os) in $g/m^3$
s= absorption cross section of the cuvette
l= intensity of the light filtered by the ozone (Os)
M = molar mass of the ozone (Os)
NA= Avogadro's number
L= path length in the cell of the cuvette
$l_0$=intensity of the light in the absence of ozone
$T_c$ = temperature of the cuvette
$T_0$ = reference ambient temperature
Prc = pressure measured
$Pr_0$ = reference ambient pressure (assumed constant)
Zcp = flow rate measured and corrective factor depending on the geometry of the cuvette

The temperature of the cuvette $T_c$ and the ambient temperature $T_0$ are considered as equal; therefore, preferably, it is not necessary to use a thermometer and the ratio is equal to 1.

Preferably, the relationship is corrected by a multiplicative corrective/calibration factor depending on the geometry of the cuvette.

Two measurements of the ozone concentration are performed in the system. A first measurement performed with the flow meter 12, the pressure sensor 14 and the cuvette 20; and a second measurement performed with the flow meter 22, the pressure sensor 23 and the cuvette 25.

For the second measurement, as very precise measurement is not necessary, only requiring to evaluate the concentration at the outlet of the catalyst 24 to determine its wear, the flow meter 22 and/or the pressure sensor 23 may be omitted.

If the ozone concentration measured downstream of the catalyst 24 exceeds a preset concentration (for example equal to 0.1 ppm), the control board 30 blocks operation of the apparatus closing the solenoid valves 13 and 26, i.e., blocking both the inflow of oxygen at the inlet and the outflow of the treated gas at the outlet, and sending a service request for replacement and/or maintenance of the catalyst 24.

According to need, the user of the apparatus selects a protocol with the relevant dosage set for the treatment chosen from a specific library displayed on the interface 31 of the control board 30.

Consequently, the user sets the ozone generator 15 as required.

If necessary, the user can manually vary the parameters set.

The apparatus collects oxygen from the oxygen cylinder 10.

The oxygen passes through the sensors and the valves and is sent to the generator 15 in which the ozone is produced. The ozone is sent to the first measuring cuvette 20. A collection valve 21, from which the user can collect the amount and dosage of ozone required/set, is located at the outlet of the cuvette. The ozone that is not used is sent to the catalyst chamber 24 to reduce the percentages of ozone still present. The gas is then dispersed into the atmosphere after further filtering, if required.

If the outflowing gas exceeds the limit established, the apparatus is blocked to allow replacement of the catalyst.

In particular, the control board 30 operates in accordance with a method that comprises the following steps.

Switching on 40 the apparatus and displaying a home page on the screen of the interface 31.

Initializing 41 the parameters of the peripherals and retrieving information from the previous sessions.

Checking 42 for the presence of problems or malfunctioning.

If present 43, indicating 44 the machine stoppage on the screen of the interface 31.

If not present 45, regulating 46 the flow rate with the proportional solenoid valve 11 and measuring the flow rate with the flow meter 12.

Checking 47 that the value measured is compatible with the value regulated.

If not compatible 48, indicating 44 the machine stoppage on the screen of the interface 31.

If compatible 49, displaying 50 the main menu on the screen of the interface 31.

Selecting 51 on the screen of the interface 31 the desired treatment from a library of preset protocols.

Automatically selecting 52 the ozone concentration according to the treatment selected.

Starting 53 the treatment.

Opening 54 the solenoid valve 13 for opening/closing the flow of oxygen to then measure the primary pressure.

Measuring 55 the primary pressure via the first pressure measuring sensor 14.

Checking 56 for the presence of anomalies.

If present 57, indicating 58 the machine stoppage on the screen of the interface 31.

If not present 59, switching on 60 the cooling fans 17.

Checking 61 for the presence of anomalies.

If present 62, indicating 63 the machine stoppage on the screen of the interface 31.

If not present 64, switching on 65 the ozone generator 15.

Checking 66 for the presence of anomalies.

If present 67 indicating 63 the machine stoppage on the screen of the interface 31.

If not present 68, measuring 69 the ozone concentration via the Beer-Lambert relationship with the values detected by the primary cuvette 20 and by the measuring devices 12 and 14.

Checking 70 if the ozone concentration reached is the same as the concentration set.

If not the same 71, repeating the step of checking 70 if the ozone concentration reached is the same as the one set.

If the same 72, collecting 73 the ozone from the collection station 21.

Filtering 74 the ozone via the catalyst 24.

Measuring 75 the secondary pressure via the second sensor 23.

Measuring 76 an ozone concentration via the Beer-Lambert relationship with the values detected by the secondary cuvette 25 and by the measuring devices 22 and 23.

Checking 77 if the ozone concentration exceeds the preset limit.

If it does 78, indicating 79 the machine stoppage on the screen of the interface 31.

If it does not 80, opening 81 the discharge solenoid valve 26.

The apparatus thus conceived is susceptible to numerous modifications and variations, all falling within the scope of the inventive concept; moreover, all details can be replaced by technically equivalent elements.

**Claims**

1. Apparatus for generating ozone comprising: an ozone generator (15); a device (21) for collecting the ozone generated; a device (24) for discharging the ozone; **characterized by** comprising a first system for measuring the ozone concentration comprising: a first measuring device (20) of a first ozone concentration; a first pressure measuring device (14) of the gas entering said first measuring device (20) of a first ozone concentration to supply a pressure measurement; a first flow rate measuring device (12) of the gas entering said first measuring device (20) of the ozone concentration to supply a flow rate measurement; a control system (30, 31) of said apparatus; said control system (30, 31) calculates a second ozone concentration based on said first ozone concentration, on said pressure measurement and on said flow rate measurement; and
by comprising a second system for measuring the ozone concentration comprising a second measuring device (25) of a third ozone concentration located downstream of said device (24) for discharging the excess ozone; said control system (30, 31) compares the value of said third ozone concentration with a preset value and if said value of said third ozone concentration exceeds said preset value it blocks said apparatus.

2. Apparatus according to claim 1, **characterized in that** said second system for measuring the ozone concentration comprises a second pressure measuring device (23) of the gas entering said second measuring device (25) of a third ozone concentration to supply a pressure measurement.

3. Apparatus according to one of the preceding claims, **characterized in that** said second system for measuring the ozone concentration comprises a second flow rate measuring device (22) of the gas entering said second measuring device (25) of the ozone concentration to supply a flow rate measurement.

4. Apparatus according to claim 1, **characterized in that** said device (24) for discharging the excess ozone comprises a catalyst.

5. Apparatus according to claim 1, **characterized by** comprising an oxygen cylinder (10) followed by a proportional solenoid valve (11) for regulating the flow rate of oxygen.

6. Apparatus according to claim 1, **characterized by** comprising a solenoid valve (13) for opening/closing the flow of oxygen located upstream of said ozone generator (15).

7. Apparatus according to claim 1, **characterized by** comprising a solenoid valve (26) for opening/closing located downstream of said second measuring device (25) of the ozone concentration.

8. Apparatus according to claim 1, **characterized in that** said first measuring device (20) of the ozone concentration comprises a cuvette associated with an LED that emits in the ultraviolet.

9. Apparatus according to claim 1, **characterized in that** said second measuring device (25) of the ozone concentration comprises a cuvette associated with an LED that emits in the ultraviolet.

10. Apparatus according to claim 1, **characterized in that** said ozone generator (15) can supply ozone in the range from 0 to 100 $\mu$g/ml.

Fig. 1

*Fig. 2A*

*Fig. 2B*

*Fig. 2C*

*Fig. 2D*

Europäisches
Patentamt

European
Patent Office

Office européen
des brevets

# EUROPEAN SEARCH REPORT

**Application Number**

EP 24 21 0307

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2014/030152 A1 (NISHIMURA SHINICHI [JP] ET AL) 30 January 2014 (2014-01-30) * paragraph [0005] - paragraphs [0013], [0072], [0081]; claims 1,4; figures 1, 5 * | 1-10 | INV. C01B13/10 |
| A | US 2020/171444 A1 (HOWARD NELSON EUGENE [US] ET AL) 4 June 2020 (2020-06-04) * paragraph [0046] - paragraphs [0047], [0083]; claim 1; figures 1,2 * | 1-10 | |

|  |
|---|
| **TECHNICAL FIELDS SEARCHED (IPC)** |
| C01B |

The present search report has been drawn up for all claims

| Place of search | Date of completion of the search | Examiner |
|---|---|---|
| Munich | 19 March 2025 | Cristescu, Ioana |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

....................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 21 0307

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2025

| Patent document cited in search report | Publication date | Patent family member(s) | Publication date |
|---|---|---|---|
| US 2014030152 A1 | 30-01-2014 | CN 103459308 A | 18-12-2013 |
| | | EP 2698343 A1 | 19-02-2014 |
| | | JP 5524201 B2 | 18-06-2014 |
| | | JP WO2012140747 A1 | 28-07-2014 |
| | | KR 20130129284 A | 27-11-2013 |
| | | TW 201240910 A | 16-10-2012 |
| | | US 2014030152 A1 | 30-01-2014 |
| | | WO 2012140747 A1 | 18-10-2012 |
| US 2020171444 A1 | 04-06-2020 | CA 3073878 A1 | 28-02-2019 |
| | | US 2020171444 A1 | 04-06-2020 |
| | | WO 2019040839 A1 | 28-02-2019 |

For more details about this annex : see Official Journal of the European Patent Office, No. 12/82

EPO FORM P0459